# EUROPEAN PATENT APPLICATION

(11) **EP 1 746 424 A1**
(43) Date of publication of application: **24.01.2007**
(21) Application number: 05720973.6
(22) Date of filing: 10.03.2005
(51) Int. Cl.: G01N 37/00, G01N 33/53

(54) **METHOD OF IMMOBILIZING PROTEIN, PROTEIN CHIP, METHOD OF IMMOBILIZING CELL AND CELL CHIP**

(30) Priority: 14.05.2004 JP 2004145565
(71) Applicant: Tohoku Techno Arch Co., Ltd., Sendai-shi, Miyagi 980-8577 (JP)
(72) Inventor: NISHIZAWA, Matsuhiko, Sendai-shi, Miyagi 980-0861 (JP); KAJI, Hirokazu, Sendai-shi, Miyagi 980-0874 (JP); MATSUE, Tomokazu, Sendai-shi, Miyagi 981-0942 (JP)
(74) Representative: Calamita, Roberto
(86) International application number: PCT/JP2005/004738
(87) International publication number: WO 2005/111630

(57) **Abstract**

It is intended to provide a novel method of immobilizing a protein and a protein chip, by which the protein can be immobilized at a high reproducibility while preventing the protein from inactivation without resort to a large-scaled apparatus and the protein can be immobilized even in a microchannel. Further, by using a cell adhesive protein as the protein to be immobilized, it is also possible to use a cell as a target and to provide a method of immobilizing a cell and a cell chip, by which a cell can be immobilized in an arbitrary region on a substrate.

## Description

### Technical Field

The invention of this application relates to a method of immobilizing a protein and a protein chip produced by the method, and also relates to a method of immobilizing a cell and a cell chip produced by the method. More particularly, the invention of this application relates to a novel method of immobilizing a protein and a protein chip by which a protein can be immobilized at a high reproducibility while preventing the protein from inactivation without resort to a large-scaled apparatus and also the protein can be immobilized even in a microchannel.

Further, the invention of this application relates to a novel method of immobilizing a cell and a cell chip by which also a cell is used as a target and can be immobilized in an arbitrary region on a substrate.

### Background Art

Now that the human genome has been deciphered, and in order to study and analyze DNA, which is a blueprint for making each protein, for the purpose of pharmacological diagnosis, drug discovery or the like utilizing the human genome data, research and development of a DNA chip has progressed. On the other hand, in order to achieve effective pharmacological diagnosis, drug discovery or the like utilizing the deciphered human genome data, only such DNA information is not sufficient. This is because proteins are substances having various higher functions in the biological activity. Therefore, it is necessary to examine the functions or information of proteins having such features.

As a tool for the purpose, a protein chip is expected, and research and development of the protein chip has been actively carried out. Further, for this protein chip, there has been a demand for development of a technique for immobilizing a protein on a substrate at a high reproducibility as its basic technique.

At present, as a technique related to the protein chip, for example, a protein chip utilizing an antigen-antibody reaction has been proposed (Document 1). In this protein chip, an antigen (or antibody) is arrayed (spotted) on a planar substrate based on a principle of an ink jet printer, and it is formed by utilizing an antigen-antibody reaction of the arrayed antigen (or antibody) and an antibody (or antigen) labeled with a fluorescent reagent or the like.

Further, a protein chip for analyzing a protein by utilizing an electrochemical technique has been proposed (Document 2). In the case of this protein chip, a substrate having a function as an electrode is used, and a protein is immobilized on the surface of this substrate in advance, and then, a sample protein labeled with an electrochemically active substance that forms a specific bond with the protein on the surface of the substrate is electrochemically detected, whereby an objective protein is analyzed.

Further, the inventor of the invention of this application has been proposed an electrochemical adhesion method as a method of immobilizing a cell on a substrate (Document 3). In this method, albumin, which is a cell non-adhesive protein, is immobilized on a substrate by the hydrophobic interaction thereby to form a cell non-adhesive surface, and an electrochemically active oxidizing species is generated with an electrode positioned near the substrate, and then the cell non-adhesive surface is modified into a cell adhesive surface, whereby a cell can be immobilized on the substrate via the cell adhesive protein. In this way, pattern immobilization of a cell can be achieved.

However, for either of the protein chip in the document 1 and the protein chip in the document 2, there were problems such that (1) it is necessary to perform immobilization in advance, therefore, the possibility that inactivation of a protein occurs during the process of immobilization, drying and storage is high; (2) it is necessary to use a relatively large-scaled apparatus; (3) a predetermined amount of a protein cannot be immobilized at a high reproducibility; and (4) immobilization in a microchannel or the like cannot be carried out; and the like.

Further, when pattern immobilization of a protein was attempted by utilizing a substrate in which a cell adhesive region was formed according to the method of the document 3, the protein could not be stably immobilized thereon. In fact, when a substrate according to the document 3 was immersed in a solution of a fluorescently labeled protein (Protein A, an antibody or the like) and fluorescence was observed, a clear fluorescent signal could not be obtained. Also in the case where a protein non-adsorptive substance other than albumin was used, there was a problem that the protein could not be stably immobilized on a substrate in a similar way.

Accordingly, in view of the above-mentioned circumstances, the invention of this application has its object to provide a novel method of immobilizing a protein and a protein chip by which the problems of the prior art are solved, a protein can be immobilized in each test and can be subjected to the test immediately after immobilization, whereby inactivation of a protein is prevented, a large-scaled apparatus is not needed, a protein can be immobilized at a high reproducibility, and moreover, a protein can be immobilized even in a microchannel.

Further, the invention of this application has its object to provide a novel method of immobilizing a cell and a cell chip, by which also a cell is used as a target and can be immobilized in an arbitrary region on a substrate by using a cell adhesive protein as the protein to be immobilized.

### Documents

Document 1: JP-A-2001-004630
Document 2: JP-A-2001-242116
Document 3: Hirokazu Kaji, Masamitsu Kanada, Daisuke Oyamatsu, Tomokazu Matsue, and Matsuhiko Nishizawa, Langmuir 2004, 20, pp. 16-19

### Disclosure of Invention

For achieving the above objects, the invention of this application provides firstly, a method of immobilizing a protein, characterized by comprising the steps of: forming a protein non-adsorptive surface by laminating a protein non-adsorptive substance with a negative charge on a substrate surface with a positive charge; locally modifying the protein non-adsorptive surface into a protein adsorptive surface; and adsorbing a protein in the locally modified region.

Further, the invention of this application provides secondly, the method of immobilizing a protein wherein the substrate surface with a positive charge is formed with a cationic polymer; thirdly, the method of immobilizing a protein wherein the cationic polymer is polyethylenimine; fourthly, the method of immobilizing a protein wherein the protein non-adsorptive substance with a negative charge is at least one substance selected from the group consisting of a glycosaminoglycan, albumin and fibrinogen; and fifthly, the method of immobilizing a protein wherein the glycosaminoglycan is at least one substance selected from the group consisting of heparin, a heparin derivative and hyaluronic acid.

The invention provides sixthly the method of immobilizing a protein wherein the modification of the protein non-adsorptive substrate surface into a protein adsorptive substrate surface is carried out with an active chemical species generated by applying an oxidation potential or an oxidation current to an electrode positioned near the substrate; seventhly, the method of immobilizing a protein wherein the active chemical species is an active halogen species generated by oxidizing a halide ion; and eighthly, the method of immobilizing a protein wherein the active halogen species is hypobromous acid (HOBr) or hypochlorous acid (HOCI).

Further, the invention provides ninthly, a method of immobilizing a protein, characterized in that protein-immobilized regions are arrayed by performing the method of immobilizing a protein according to any one of the above first to eighth inventions in a sequential manner; tenthly, the method of immobilizing a protein wherein the protein-immobilized regions are established by arraying and immobilizing a protein non-adsorptive substrate surface modifying agent, which is insensitive to the active chemical species, on the substrate surface; and eleventhly, the method of immobilizing a protein wherein the substrate surface modifying agent is at least either of a polyethylene glycol polymer and a methacryloyloxyethyl phosphorylcholine polymer.

Further, the invention of this application provides twelfthly, a protein chip which is produced by the method of immobilizing a protein according to any one of the above first to eleventh inventions, characterized in that a protein non-adsorptive substrate surface is locally modified into a protein adsorptive substrate surface and a protein is locally immobilized; thirteenthly, the protein chip wherein a microchannel and an electrode system are embedded in the substrate; and fourteenthly, the protein chip wherein the embedded electrode system is a bipolar electrode system comprising one pair of electrodes, and a counter electrode is made of platinum.

Further, the invention of this application provides fifteenthly, the protein chip wherein the locally immobilized protein is an antibody; sixteenthly the protein chip wherein the locally immobilized protein is Protein A or Protein G, and by binding an antibody to this Protein A or Protein G, the antibody is immobilized on the substrate surface; and seventeenthly the protein chip wherein the locally immobilized protein is an enzyme.

The invention provides eighteenthly the protein chip wherein the locally immobilized protein is a cell adhesive protein; and nineteenthly, the protein chip wherein the cell adhesive protein is at least one protein selected from the group consisting of fibronectin, collagen and laminin.

Further, the invention of this application provides twentiethly, a method of immobilizing a cell, characterized by allowing a cell to adhere on the protein chip according to the above eighteenth or nineteenth invention; and twenty-firstly, a cell chip, which is produced by the method of immobilizing a cell according to the above twentieth invention, characterized in that a cell is locally immobilized thereon.

### Brief Description of Drawings

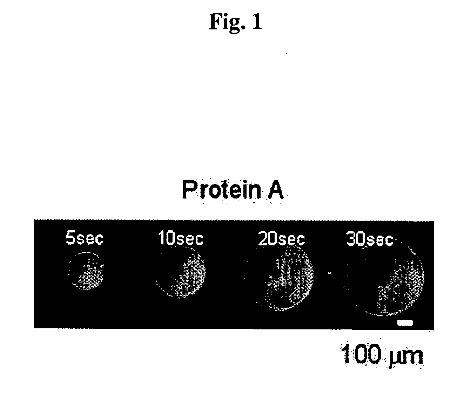

Fig. 1 is a photograph showing that as a protein, Protein A was locally immobilized by an electrochemical treatment according to the invention of this application and the protein-immobilized area size can be controlled by the time for which an electric potential is applied (for 5 sec, 10 sec, 20 sec and 30 sec).

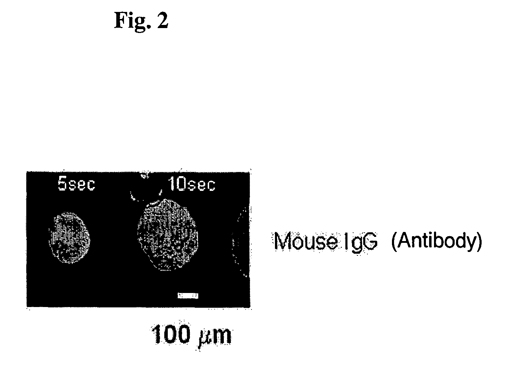

Fig. 2 is a photograph showing a state in which mouse IgG (antibody) was immobilized as a protein in the same method as in Fig. 1.

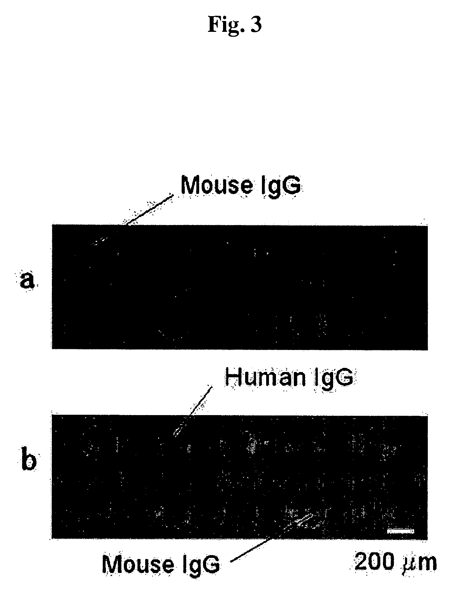

Fig. 3 is a photograph showing a state in which two types of antibodies were bound to Protein A locally immobilized on a substrate. In Fig. 3(a), mouse IgG was bound as the antibody, and in Fig. 3(b), human IgG was bound as the antibody.

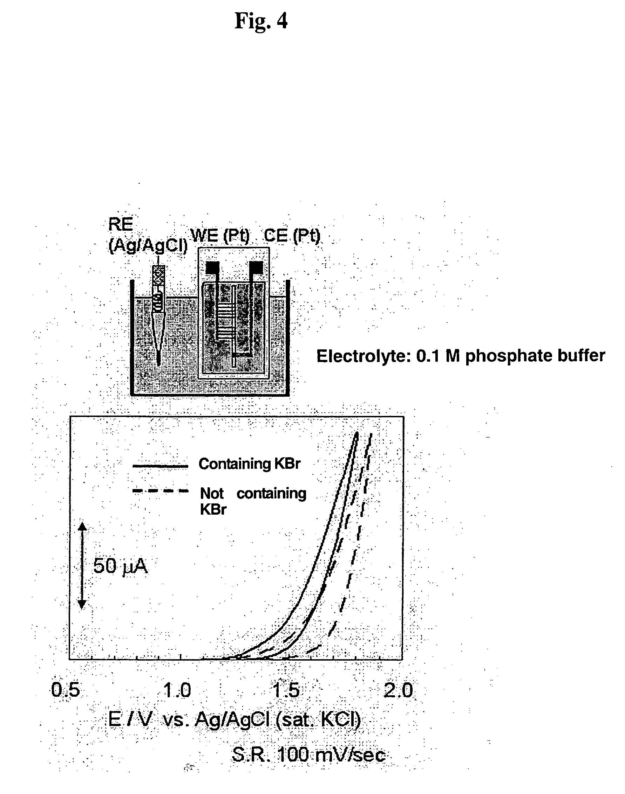

Fig. 4 is a schematic diagram of an experimental system in which one electrode of the substrate electrodes was used as a working electrode and the other electrode was used as a counter electrode, and an additionally inserted silver-silver chloride electrode was used as a reference electrode, and a view showing a cyclic voltammogram (CV) obtained using this system.

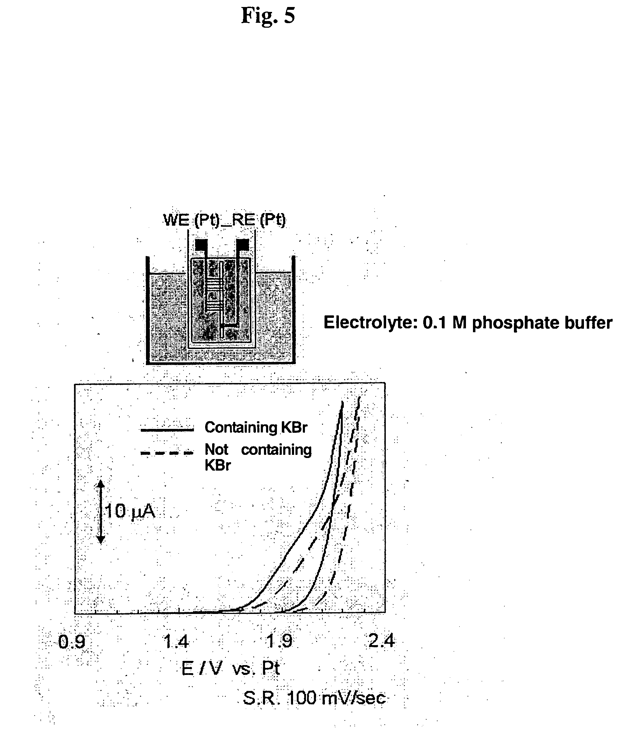

Fig. 5 is a schematic diagram of an experimental system in which one electrode of the substrate electrodes was used as a working electrode and the other electrode was used as a reference electrode (also acted as a counter electrode), and a view showing a cyclic voltammogram (CV) obtained using this system.

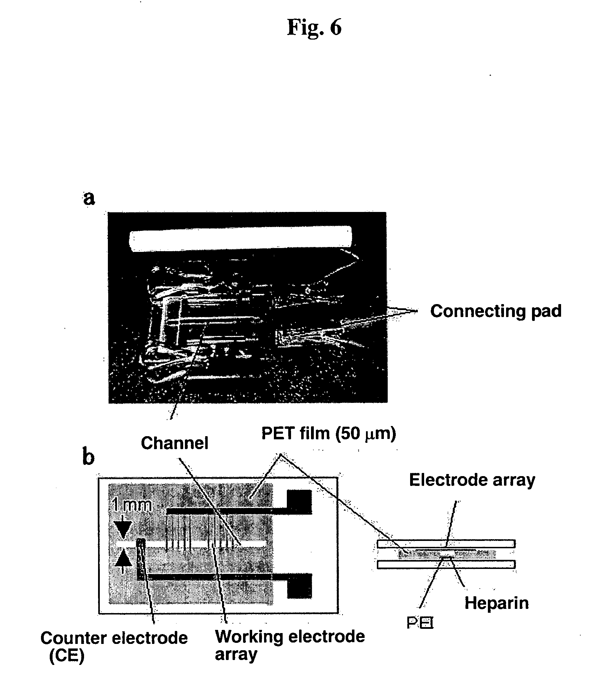

Fig. 6 is a view schematically illustrating a microchannel composed of the electrode substrate shown in Fig. 5. Fig. 6(a) is a photograph showing the whole microchannel chip, and Fig. 6(b) is a plan view and a cross-sectional view schematically illustrating the microchannel chip.

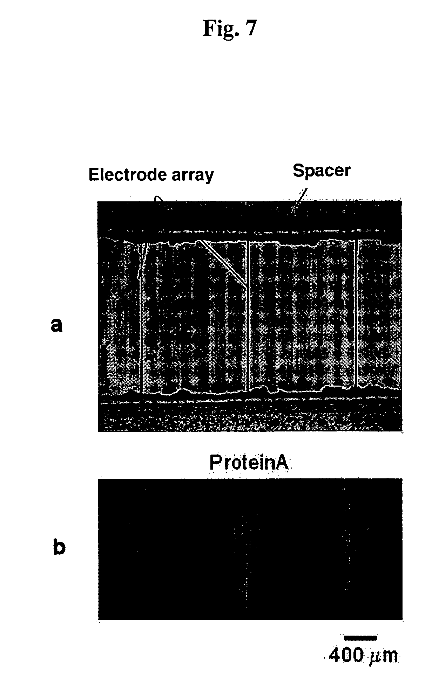

Fig. 7 is a photograph showing a state in which a protein was locally immobilized in a channel using a microchannel in which the electrode system shown in Fig. 6 was embedded. Fig. 7(a) shows electrodes positioned in the upper wall of the channel, and Fig. 7(b) shows the protein immobilized on the bottom wall of the channel.

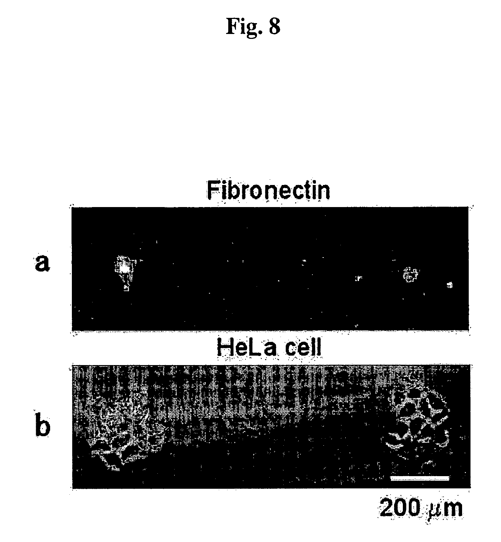

Fig. 8 is a photograph showing a state in which a culture cell (HeLa cell) was locally immobilized on a substrate. Fig. 8(a) is a photograph in which fibronectin was fluorescently labeled so as be visualized, and Fig. 8(b) is a phase-contrast micrograph showing the results of culturing HeLa cell on this substrate.

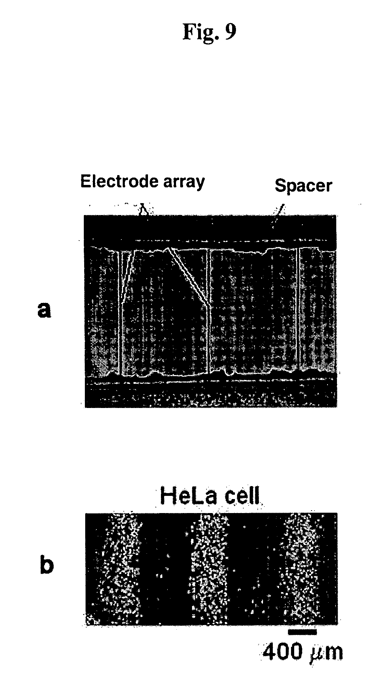

Fig. 9 is a photograph showing a state in which a culture cell (HeLa cell) was locally cultured in a channel using a microchannel in which the electrodes shown in Fig. 6 were embedded. Fig. 9(a) shows electrodes positioned in the upper wall of the channel, and Fig. 9(b) shows the cell adhering to the bottom wall of the channel.

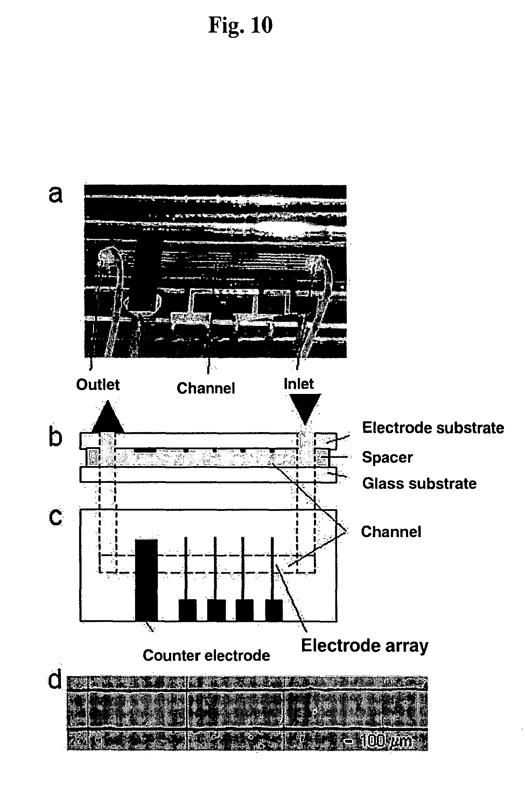

Fig. 10 is a view illustrating a microchannel chip according to the invention of this application. Fig. 10(a) is a photograph showing the whole microchannel chip, Fig. 10(b) is a cross-sectional view schematically illustrating the microchannel chip, Fig. 10(c) is a plan view schematically illustrating the microchannel chip, and Fig. 10(d) is a plan view illustrating an electrode array of the microchannel chip.

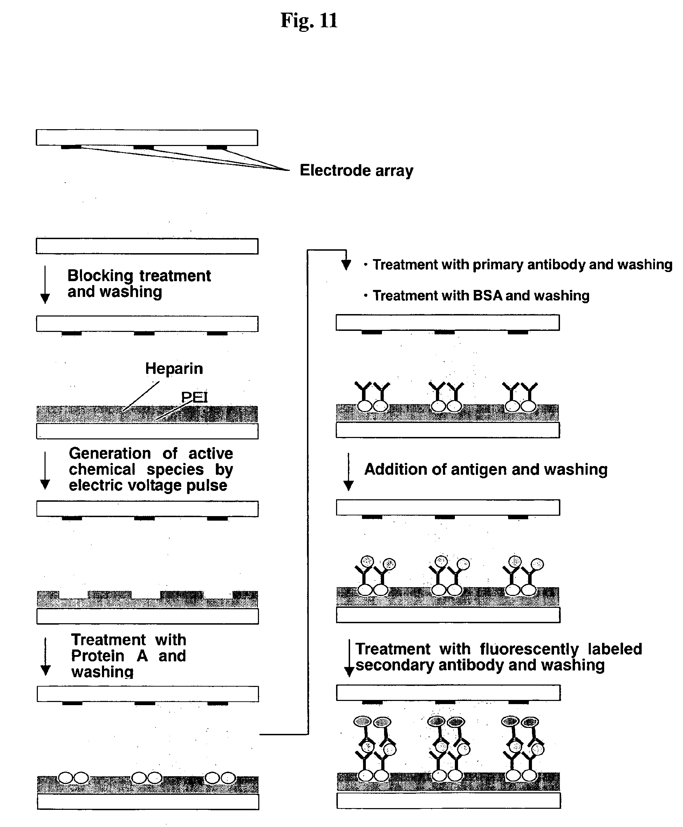

Fig. 11 is a schematic diagram showing a step of a sandwich immunoassay using a microchannel chip according to the invention of this application.

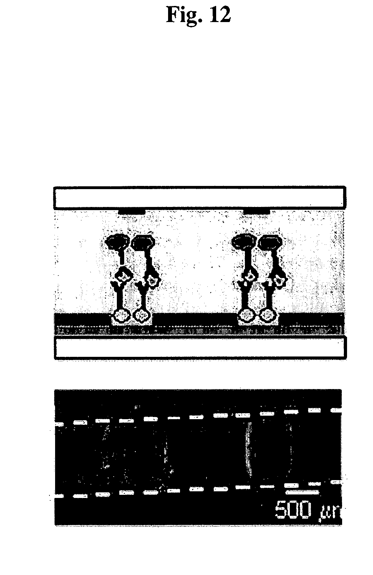

Fig. 12 is schematic view showing the results of fluorescence observation in the sandwich immunoassay in Fig. 11 and a photograph of the observation thereof.

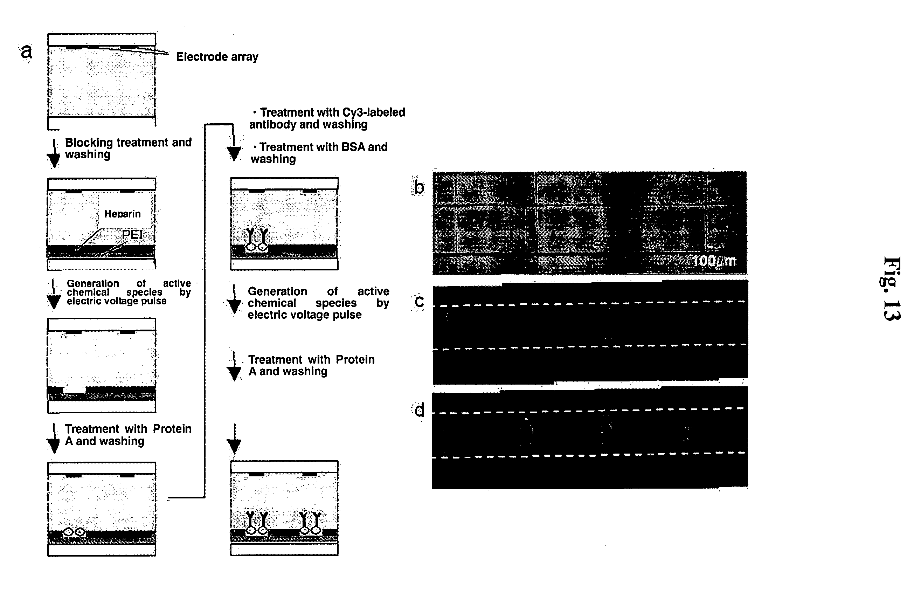

Fig. 13 is a view showing a mode of multi-antibody patterning on a microchannel chip according to the invention of this application. Fig. 13(a) is a schematic diagram showing a step thereof, Fig. 13(b) is a plan view photograph showing an electrode array (ceiling portion of the channel), Fig. 13(c) is a photograph showing a mode of reaction of a first type of antibody (Cy3-labeled mouse IgG), and Fig. 13(d) is a photograph showing a mode of reaction of a second type of antibody (Cy2-labeled human IgG) along with the first type of antibody.

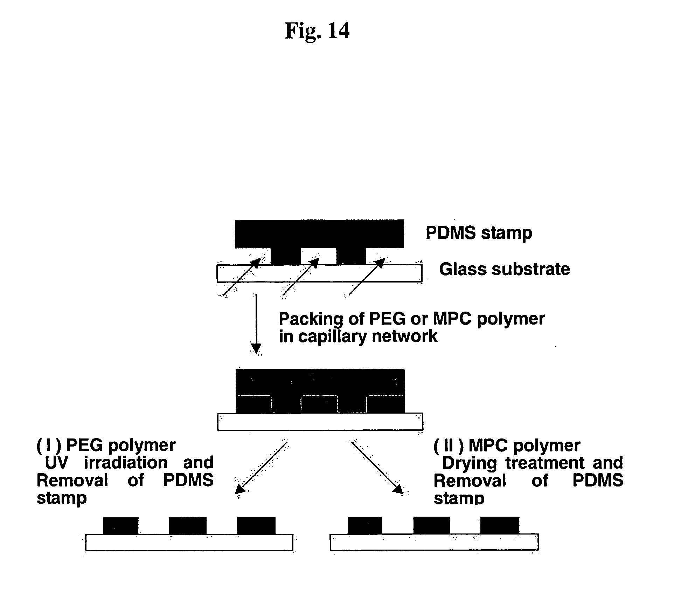

Fig. 14 is a schematic diagram showing a step of patterning with a substrate surface modifying agent (PEG polymer or MPC polymer) according to the invention of this application.

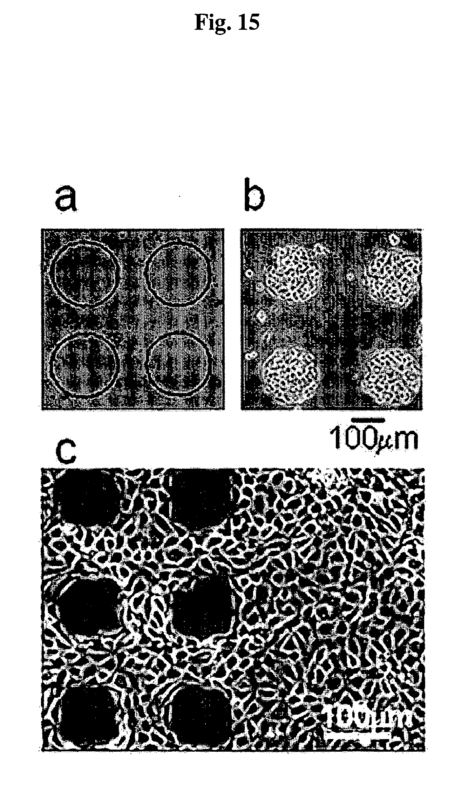

Fig. 15 is a photograph showing a mode in which a cell was cultured on a glass substrate patterned with PEG polymer or MPC polymer, which is a substrate surface modifying agent. Fig. 15(a) shows the glass substrate patterned with PEG polymer, Fig. 15 (b) shows a mode of HeLa cell cultured for 3 days on the glass substrate patterned with PEG polymer, and Fig. 15(c) shows a mode of a bovine aortic vascular endothelial cell cultured for 1 month on the substrate patterned with MPC polymer.

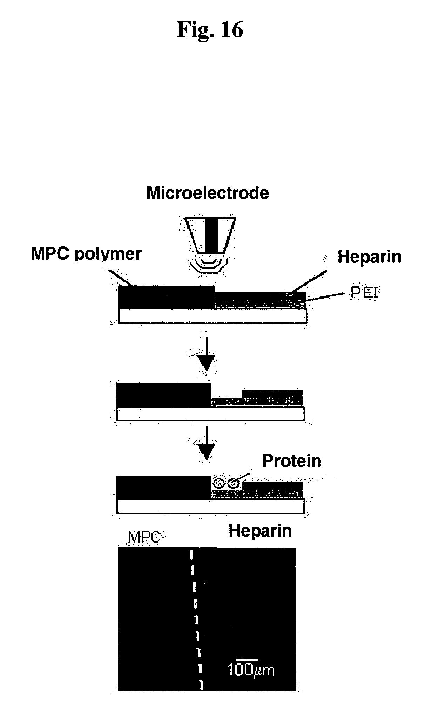

Fig. 16 is a schematic diagram and a photograph showing a mode of a resistance test for MPC polymer, which is a substrate surface modifying agent, against an active oxidizing species (hypobromous acid (HOBr)).

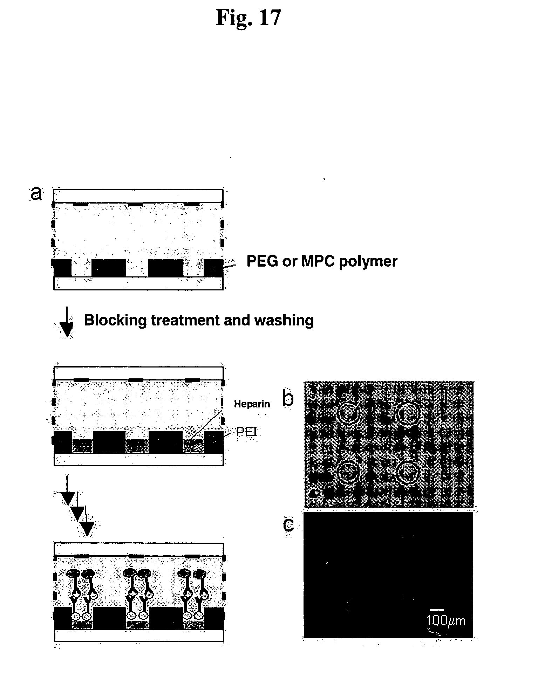

Fig. 17 is a view showing a sandwich immunoassay using a microchannel chip subjected to a treatment with a substrate surface modifying agent (PEG polymer or MPC polymer) according to the invention of this application. Fig. 17(a) is a schematic diagram showing a step of the sandwich immunoassay, Fig. 17(b) is a plan view photograph of a channel substrate patterned with MPC polymer, and Fig. 17(c) is a photograph showing a mode in which a protein immobilized only on a specified region was confirmed by fluorescence observation.

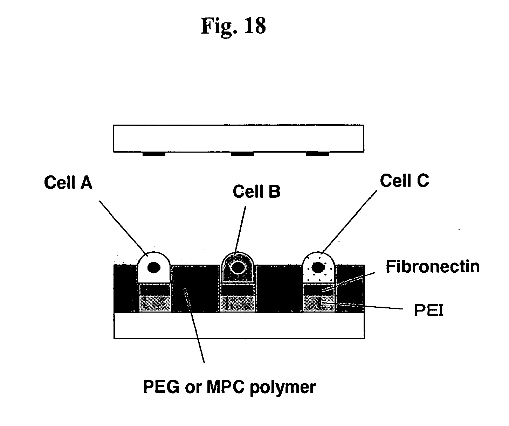

Fig. 18 is a schematic view showing a mode of multi-cell patterning culture using a substrate surface modifying agent.

### Best Mode for Carrying Out the Invention

The invention of this application has characteristic features as described above, however, hereinafter an embodiment thereof will be described in detail.

The invention of this application is characterized by being a method of immobilizing a protein in which a local area of a protein non-adsorptive substrate surface is modified thereby to adsorb a protein thereon and a protein chip produced by this method. Specifically, this method of immobilizing a protein is characterized by comprising the steps of: forming a protein non-adsorptive surface by laminating a protein non-adsorptive substance with a negative charge on a substrate surface with a positive charge; locally modifying the protein non-adsorptive surface into a protein adsorptive surface; and adsorbing a protein in the locally modified region.

Here, "locally" means that it generally has a size of submicron to several hundreds of microns. By modifying a protein non-adsorptive substrate surface into a protein adsorptive substrate surface by limiting the surface to be modified to an arbitrary area of the substrate, a region in which an arbitrary protein can be adsorbed on the substrate can be patterned.

Further, in order for the substrate surface to have a positive charge, it is preferably formed by coating the substrate surface with a cationic polymer. As the cationic polymer, polyethylenimine, polyvinylamine, polyallylamine, polyornithine, polylysine or the like is preferably used.

In the invention of this application, the protein non-adsorptive substance with a negative charge to be immobilized on the substrate surface for making the substrate surface non-adsorptive to a protein is not particularly limited as long as it exerts an activity to prevent a protein from being adsorbed on the substrate. For example, as a specific example, albumin, fibrinogen, a glycosaminoglycan or the like can be used. Further, as the glycosaminoglycan, for example, heparin, a heparin derivative, hyaluronic acid or the like can be used.

That is, in the invention of this application, since a protein generally has a negative charge, an area where a protein is desired to be adsorbed and immobilized is modified into a protein adhesive surface by exposing the substrate surface with a positive charge (for example, by formation with a cationic polymer), and an area where a protein is not desired to be adsorbed or immobilized is formed as a protein non-adsorptive surface. In this way, a protein with a negative charge can be adsorbed and immobilized on the substrate with a positive charge by the electrostatic interaction. On the other hand, it was found that a protein non-adsorptive surface cannot be formed only by allowing the surface to have a negative charge. Further, it can also be taken into account that adsorption of a protein is inhibited by not only electrostatic repulsion but also a biological activity inherent in the protein non-adsorptive substance.

With regard to the modification of a protein non-adsorptive substrate surface into a protein adsorptive substrate surface in the invention of this application, for example, an electrode such as a microelectrode is positioned near the substrate, and an oxidation potential or an oxidation current is applied to this electrode, whereby an electrochemically active chemical species is locally generated, and by using this active chemical species, a region where a protein can be adsorbed can be formed. That is, by applying an oxidation potential or an oxidation current (or it is sometimes called an oxidation pulse if the applying time is short) to an electrode in an arbitrary area in the substrate, an active chemical species is generated, and only this area can be altered (modified) into a protein adsorptive area. Thus, it becomes possible to adsorb a protein on the substrate. The size of the electrode in this case is small, therefore, a series of these reactions are carried out locally as described above. Of course, this electrode can be positioned at an arbitrary place, and further, a plurality of electrodes can be positioned such that they are arrayed. Further, its displacement, operation or the like can be controlled by a computer connected to the electrode. As a substance that is modified from protein non-adsorptive to protein adsorptive by this active chemical species, for example, as described above, albumin, heparin, a heparin derivative, fibrinogen, hyaluronic acid and the like can be exemplified.

Incidentally, as the active chemical species, an active halogen species generated by oxidizing a halide ion is preferred. The halide ion is an ion of a halogen element belonging to the group 17 (group 7B) of the periodic table, and any halide ion can be used. Specifically, it is fluorine (F), chlorine (Cl), bromine (Br), iodine (I) or astatine (At). It is more preferred that the active halogen species is either of hypobromous acid (HOBr) and hypochlorous acid (HOCl).

Further, in the invention of this application, by performing the method of immobilizing a protein as described above in a sequential manner, it is also possible to array the protein-immobilized regions in a desired pattern. In particular, as described above, by positioning a plurality of electrodes in a desired pattern and sequentially applying an oxidation potential or an oxidation current to the electrodes, a plurality of and arbitrary proteins can be adsorbed on the substrate. At this time, a plurality of the same proteins may be adsorbed thereon, or a plurality of different types of proteins may be adsorbed thereon.

Further, at this time, in the case where the active chemical species is used, by arraying and immobilizing a protein non-adsorptive substrate surface modifying agent, which is insensitive to the active oxidizing species (i.e., a substrate surface modifying agent which is not modified into protein adsorptive by the active oxidizing species), on the substrate surface, a protein can be immobilized only in a region where albumin, heparin, a heparin derivative, fibrinogen, hyaluronic acid or the like, which is modified from protein non-adsorptive to protein adsorptive by the active oxidizing species (sensitive to the active oxidizing species), has been immobilized independent of the conditions of generating the active oxidizing species (i.e., conditions of the oxidation potential or oxidation current), more efficiently, accurately and reproducibly.

Incidentally, it is preferred that the protein non-adsorptive substrate surface modifying agent, which is insensitive to the active oxidizing species is at least either of a polyethylene glycol polymer (PEG polymer) and a methacryloyloxyethyl phosphorylcholine polymer (MPC polymer). Further, these substrate surface modifying agents are generally stable for a long period of time.

Specifically, with the use of a substrate on which a protein has already been adsorbed, an oxidation potential (oxidation pulse or the like) is applied to an electrode positioned at an arbitrary place where a protein is not adsorbed, whereby an active chemical species is generated. This active chemical species modifies the arbitrary place (local area) in the substrate into a protein adsorptive substrate surface again, and by adsorbing a new protein thereon, the protein can be adsorbed on the substrate in an arbitrary pattern. As described above, the type of the protein to be newly adsorbed at this time is not particularly limited, and it may be the same type or a different type of the protein adsorbed previously. That is, the type of the protein can be appropriately adopted in accordance with the purpose of the study, experiment or the like.

For example, in the invention of this application, a protein chip is produced using an antibody as the protein to be adsorbed (immobilized) on the substrate, and this protein chip can be applied to an immunoassay as an antibody chip. Further, in the step of producing such an antibody chip, after Protein A or Protein G is adsorbed, an antibody can be bound thereto. Because this Protein A or Protein G is bound to the constant region (Fc region) of an antibody, an antibody can be effectively aligned, whereby the sensitivity and accuracy of an immunoassay using the produced antibody chip can be improved.

Further, by performing the immobilization method as described above in a sequential manner, a protein chip in which the constant regions of antibodies are arrayed can also be produced.

By using a cell adhesive protein such as fibronectin, collagen or laminin as the protein to be adsorbed on the substrate, the invention of this application can be applied to production of a cell chip in which a cell is immobilized. At this time, by using the substrate surface modifying agent in combination, it can be expected that the cell chip can be applied to multi-cell patterning culture in which various cells are cultured or long-term pattern culture.

The "cell (culture cell)" that can be used in the invention of this application may be a cell of any origin. For example, a plant cell, an insect cell, an animal cell or the like can be used, or it may be a fused cell obtained by fusing cells of heterologous origins with each other or fusing a cell with a noncellular substance such as a collagen gel membrane, cocoon filament or nylon mesh.

Of course, the culture cell may be a primary cell or an established cell line. In particular, it is preferably an animal cell. As the primary cell in animal cells, a cell derived from chick embryo (PSG), a primary rat cardiomyocyte, a primary rat hepatocyte, a primary murine bone marrow cell, a primary porcine hepatocyte, a bovine vascular endothelial cell, a primary human umbilical cord blood cell, a primary human bone marrow hematopoietic cell, a primary human neuron such as a dorsal root ganglion cell (DRG) and the like can be exemplified. Further, as the established cell line, a CHO cell derived from a Chinese hamster ovary cell, a HeLa cell derived from human uterine cancer, a Huh7 cell or HepG2 cell derived from human liver cancer, a neuron such as a dorsal root ganglion cell (DRG), a cardiomyocyte, endothelial cell or the like can be used. Further, a cell obtained by introducing a plasmid into any of these cells or genetic engineering such as virus infection can also be used in the invention of this application.

Further, these cells may be adhesive cells or floating cells, however, it is preferred that these cells are adhesive cells because an effect of the invention of this application can be remarkably obtained.

Further, by forming a protein chip using an enzyme as the protein to be adsorbed on the substrate, the invention of this application can also be applied to a biochemical analysis chip utilizing an enzyme reaction. The enzyme is not particularly limited, and for example, a variety of enzymes such as peroxidase, tyrosine kinase, dehydrogenases for a variety of saccharides including glucose can be used.

In the invention of this application, an electrode system composed of a working electrode and a counter electrode, etc., which is necessary for controlling and advancing an electrochemical reaction for generating the active chemical species, can be embedded in a microchannel chip, and a protein can be efficiently immobilized also in the microchannel.

A material to be used as the substrate in the invention of this application is not particularly limited and various types of substrates, for example, not to mention a cationic polymer per se, substrates obtained by coating a semiconductor, a glass plate, a plastic plate and a metal thin film, and the like can be used.

According to the invention of this application as described above, inactivation of a protein is prevented, and an electrochemical system which is small-scaled and inexpensive compared with a conventional one is used, therefore a large-scaled apparatus is not necessary, and a protein can be immobilized at a high reproducibility, and moreover, a protein can be immobilized even in a microchannel.

In this way, by enabling a protein to be immobilized on a substrate promptly and easily, it can be provided as an innovative tool to a wide variety of fields including pharmacological diagnosis, drug discovery, analysis and test for food or environment, medical engineering, sensor engineering and the like, whereby great effect can be brought about industrially and economically. Further, by using a cell adhesive protein as the protein to be immobilized, it also becomes possible to immobilize a cell in an arbitrary region on a substrate, whereby its application range will be expanded.

Hereinafter, the invention of this application will be described in more detail by describing Examples. Of course, the invention is by no means limited to the following examples.

### Examples

### Example 1: Use of electrochemical treatment

### <I> In the case where heparin was used as a protein non-adsorptive substance

### (1) Pretreatment of substrate

A glass plate was used as a substrate. This substrate was washed and immersed in an aqueous solution of polyethylenimine (PEI) (10 mg/mL) for 2 hours, whereby a PEI layer was formed. Then, the substrate was immersed in an aqueous solution of heparin (2 mg/mL) for 30 minutes, whereby heparin was immobilized on the substrate surface by the electrostatic interaction and the substrate surface was made non-adsorptive to a protein.

### (2) Electrochemical treatment

In a phosphate buffer containing 25 mM KBr, a Pt disk microelectrode (electrode diameter: 20 µm) to which bromide ion oxidation potential (1.7 V vs. Ag/AgCl) was applied was positioned near the substrate, and an active halogen species was generated. By this treatment, the PEI layer is exposed in the substrate surface, and functions as a linker layer for a protein which will be immobilized later. Here, the Pt disk microelectrode was a working electrode (WE), and the treatment was carried out by connecting a potentiostat to a three-electrode system in which an Ag/AgCl electrode was used as a reference electrode (RE) and a platinum plate was used as a counter electrode (CE).

### (3) Immobilization of protein

Then, the substrate was immersed for 20 minutes in a solution of Protein A (0.025 mg/mL) fluorescently labeled according to a known method. After washing the substrate, it was confirmed that Protein A was locally immobilized by observing the fluorescence. Incidentally, as shown in Fig. 1, an area where Protein A is adsorbed (protein-immobilized area size) can be controlled by the time for which an electric potential is applied (for 5 sec, 10 sec, 20 sec and 30 sec).

As the protein to be adsorbed (immobilized) on the substrate, other than the above-mentioned Protein A, a variety of antibodies and a variety of cell adhesive proteins could be locally immobilized. For example, as shown in Fig. 2, mouse IgG (antibody) could be locally immobilized, and in the same manner as in Fig. 1, it could be confirmed that the adsorbed area is expanded in proportion to the length of the time for which an electric potential is applied.

### <II> In the case where albumin was used as a protein non-adsorptive substance

Immobilization of a protein on a substrate was carried out in accordance with the same experimental procedure as in the above Example 1.

Although the results are not shown in the drawing, the protein could be immobilized in the same manner as in Example 1, and it could also be confirmed that an area where Protein A is adsorbed (protein-immobilized area size) can be controlled by the time for which an electric potential is applied.

### Example 2: Patterning of a plurality of types of proteins

The operations of (2) and (3) in Example 1 were repeated twice, and two types of proteins were immobilized on the same substrate. Specifically, first, fluorescently labeled mouse IgG was immobilized via locally immobilized Protein A (immersion in a solution of mouse IgG (0.025 mg/mL) for 20 minutes), then, the substrate was immersed in a solution of bovine serum albumin (2 mg/mL) for 30 minutes. Subsequently, Protein A was locally immobilized again, and then, fluorescently labeled human IgG was immobilized (immersion in a solution of human IgG (0.025 mg/mL) for 20 minutes).

The results are as shown in Figs. 3(a) and 3(b), and mouse IgG and human IgG could be immobilized on the same substrate, respectively.

### Example 3: Generation of active halogen species using substrate integrated with electrode system

In Examples 1 and 2, an electrochemical treatment was carried out using a Pt disc microelectrode (electrode diameter: 20 µm) juxtaposed to the substrate surface. On the other hand, a method in which an electrode is prepared on another substrate in advance and is faced using a spacer to a glass substrate subjected to a pretreatment in the same manner as in Example 1 (1), and an electrochemical treatment is carried out is desired in some cases from a practical point of view. In addition, generally, it is preferred that a reference electrode which is prepared separately is also loaded on an electrode substrate. In the light of this, as Example 3, it was shown that an active halogen species can be electrolytically generated using only a substrate patterned in which a pair of platinum electrodes were patterned.

First, as shown in Fig. 4, one electrode of the substrate electrodes was used as a working electrode (WE) and the other electrode was used as a counter electrode (CE), and a separately inserted silver-silver chloride electrode (Ag/AgCl) was used as a reference electrode (RE), and a cyclic voltammometry (CV) was carried out in a phosphate buffer (0.1 M, pH 7.5) containing 0.1 M KCl. In this Fig. 4, in the case of the presence of 25 mM KBr, as indicated by the solid line, an oxidation current in which contribution of bromide ion oxidation is dominant was observed. Meanwhile, in the case of the absence of KBr, as indicated by the dashed line, an oxidation current in which contribution of chloride ion oxidation is dominant was observed.

Subsequently, as shown in Fig. 5, one electrode of the substrate electrodes was used as a working electrode (WE) and the other electrode was used as a reference electrode (RE) (also acted as a counter electrode), and CV was carried out in the same solution. In the case of the presence of KBr, an oxidation current in which contribution of bromide ion oxidation is dominant was observed, and in the case of the absence of KBr, an oxidation current in which contribution of chloride ion oxidation is dominant was observed. The obtained CV form was the same as in the case of Fig. 4. That is, even in the case where a reference electrode was not prepared outside separately, an oxidation reaction of a halide ion could be controlled in a two-electrode electrochemical system in which the platinum electrode on the substrate was used as a reference electrode.

The results indicate that the whole electrode system can be integrated on a small substrate, and it has an effect of considerably simplifying the protein chip of the invention of this application and the structure of a device associated with the protein chip.

### Example 4: Immobilization of protein in microchannel

In a substrate in which a microchannel was formed in this Example, as shown in Fig. 6, a channel was formed by being sandwiched between the electrode substrate of Example 3 and a glass substrate on which heparin was immobilized in the same manner as in Example 1 (1) via a PET film spacer.

In this channel, a phosphate buffer containing 25 mM KBr was packed, and bromide ion oxidation potential (2.2 V vs. Pt) was applied to the working electrode, whereby a protein adsorptive region was formed. Subsequently, a solution of fluorescently labeled Protein A (0.025 mg/mL) was packed therein for 10 minutes, and washing was carried out, and then, observation was carried out with a fluorescence microscope.

The results are as shown in Fig. 7, and on the heparin- immobilized substrate, a protein (Protein A) could be immobilized corresponding to the electrode position in the electrode substrate.

A protein chip combined with a microchannel is a preferred embodiment which requires a reduced amount of a solution to be used and has an effect of improving the continuity and reproducibility of the operation. This Example implemented immobilization of a protein in a local area in a microchannel for the first time, which had been extremely difficult so far, and has an effect of solving the problem of a protein chip.

### Example 5: Patterning of cell

The basic operations are the same as the operations in Example 1 (2) and (3). As the protein to adhere to the substrate surface locally, fibronectin, which is a cell adhesive protein, was used and patterning adhesion thereof was carried out.

In an area in which patterning adhesion of fibronectin was carried out, as a culture cell, HeLa cell was inoculated and cultured. As a result, as shown in Fig. 8, adhesion of HeLa cell could be selectively induced only on this pattern of fibronectin. In Fig. 8(a), fibronectin was fluorescently labeled so as be visualized, and Fig. 8(b) is a phase-contrast micrograph showing the results of culturing HeLa cell on this substrate.

Further, although it is not shown in the drawing, it was also possible to carry out patterning of other culture cells, for example, a neuron, a cardiomyocyte and an adhesive cell such as an endothelial cell by the same method.

### Example 6: Patterning of cell in microchannel

As shown in Fig. 9, the same apparatus as the apparatus used in Example 4, that is, an apparatus in which a channel was formed by being sandwiched between an electrode substrate and a glass substrate on which heparin was immobilized via a PET film spacer was used. After fibronectin, which is a cell adhesive protein, was immobilized, as a culture cell, HeLa cell was inoculated and cultured.

The results are as shown in Fig. 9. As shown in Fig. 9, the cell (HeLa cell) could adhere to a local area in the microchannel of the substrate. From these results, considering that as for the application of a cell chip to such as cell diagnosis, "in situ immobilization" as this result is important, the cell chip of the invention of this application can be adequately utilized also for cell diagnosis or the like. Further, the cell chip of the invention of this application can also be utilized as a tool for such as studying and analyzing the intercellular interaction such as binding of a tumor cell to T cell from the viewpoint of the applied study of such as basic biology or drug discovery.

### Example 7: Immobilization of protein on substrate with microchannel (microchannel chip)

### (1) Production of microchannel chip

As shown in Fig. 10, Pt was patterned on a glass substrate using a series of microprocessing techniques, whereby an electrode substrate in which an electrode was embedded was produced. A channel was formed by being sandwiched between this electrode substrate and the glass substrate via a spacer (silicon rubber with a thickness of 50 µm). Feeding of liquid into this channel was carried out by connecting a narrow tube of an inlet to a reservoir in which a desired solution was placed and sucking at an outlet. In the reservoir of the inlet, a silver-silver chloride electrode was installed as a reference electrode.

### (2) Immobilization of protein in channel and immunoassay

### <A> Patterning of antibody

In the produced microchannel chip, a sandwich immunoassay was carried out in accordance with the following steps as shown in Fig. 11.

An aqueous solution of polyethylenimine (PEI) (5 mg/mL) was introduced into the channel and incubation was carried out for 30 minutes.

Then, an aqueous solution of heparin (2 mg/mL) was introduced into the channel and incubation was carried out for 20 minutes, whereby heparin was immobilized on the substrate surface by the electrostatic interaction.

Then, a phosphate buffer containing 25 mM KBr was packed in the channel and bromide ion oxidation potential (1.7 V vs. Ag/AgCl) was applied to the working electrode for 5 seconds or 10 seconds, whereby the PEI/heparin layer immobilized on the substrate was modified.

Then, a solution of Protein A (25 µg/mL) was packed in the channel and incubation was carried out for 30 minutes, whereby Protein A was immobilized on the substrate.

Then, a solution of a primary antibody (goat-derived anti-mouse IgG: 25 µg/mL) was introduced into the channel and incubation was carried out for 30 minutes, whereby the antibody was immobilized on the substrate.

Then, a solution of bovine serum albumin (BSA) (5 mg/mL) was introduced into the channel and incubation was carried out for 30 minutes, whereby blocking was achieved.

Then, a solution of an antigen (mouse IgG: 25 µg/mL) was introduced into the channel and incubation was carried out for 30 minutes.

Then, a solution of a fluorescently labeled secondary antibody (FITC-labeled goat-derived anti-mouse IgG: 25 µg/mL) was introduced into the channel and incubation was carried out for 30 minutes, and then, fluorescence was observed.

The results are as shown in Fig. 12, and an antibody reaction could be confirmed only in the area in which heparin was pattern-immobilized (patterned). Further, it could be confirmed that the reaction intensity is improved depending on the reaction time.

### <B> Patterning of multi-antibody

In the produced microchannel chip, a sandwich immunoassay was carried out in accordance with the following steps as shown in Fig. 13(a). Incidentally, the operation procedure is basically the same as the above <A>.

An aqueous solution of polyethylenimine (PEI) (5 mg/mL) was introduced into the channel and incubation was carried out for 30 minutes.

Then, an aqueous solution of heparin (2 mg/mL) was introduced into the channel and incubation was carried out for 20 minutes, whereby heparin was immobilized on the substrate surface by the electrostatic interaction.

Then, a phosphate buffer containing 25 mM KBr was packed in the channel and bromide ion oxidation potential (1.7 V vs. Ag/AgCl) was applied to the working electrode for 10 seconds, whereby the PEI/heparin layer immobilized on the substrate was modified.

Then, a solution of Protein A (25 µg/mL) was packed in the channel and incubation was carried out for 30 minutes, whereby Protein A was immobilized on the substrate.

Then, a solution of a first type of antibody (Cy3-labeled mouse: 25 µg/mL) was introduced into the channel and incubation was carried out for 30 minutes, whereby the antibody was immobilized on the substrate.

Then, a solution of bovine serum albumin (BSA) (5 mg/mL) was introduced into the channel and incubation was carried out for 30 minutes, whereby blocking was achieved.

Then, the operation of modification of the PEI/heparin layer was carried out, and then the operation of immobilization of Protein A on the substrate was also carried out again.

Then, a second type of antibody (Cy2-labeled human IgG: 25 µg/mL) was introduced into the channel and incubation was carried out for 30 minutes, and then, fluorescence was observed.

As a result, as shown in Figs. 13(b), (c) and (d), an antibody reaction could be confirmed only in the area in which heparin was pattern-immobilized (patterned) for either of the first type of antibody (Cy3-labeled mouse) and the second type of antibody (Cy2-labeled human IgG).

### Example 8: Immobilization of protein using substrate surface modifying agent

Polyethylene glycol (PEG) or MPC polymer, which is a substrate surface modifying agent, is a surface modifying agent with long term stability, which is resistant to hypobromous acid (HOBr), which is an active oxidizing species.

A method of patterning with PEG or MPC polymer, which is the substrate surface modifying agent, will be described. Incidentally, a polydimethylsiloxane (PDMS) stamp was produced using a glass substrate obtained by patterning a photoresist (film thickness: 9 µm) by photolithography as a template.

As shown in Fig. 14, this PDMS stamp was placed on a glass substrate, and a solution of PEG (poly(ethylene glycol) dimethacrylate) or MPC polymer was poured into the gap between the concave portions and the substrate surface utilizing the capillary phenomenon. Here, as the solution of PEG, a solution mixture of 99.5 wt % of poly(ethylene glycol) dimethacrylate with an average molecular weight of 550 and 0.5 wt % of 2-hydroxy-2-methylpropiophenone of a photopolymerization initiator was used. Further, as the MPC polymer, an ethanol solution of MPC polymer (5 wt %) was used.

In the case where the solution of PEG was poured, after the polymer was cured by UV irradiation (365 nm, 15 mW/cm²; 20 sec), the PDMS stamp was removed. In the case of the solution of MPC polymer, after the substrate was dried for 20 minutes, the stamp was removed.

Fig. 15 is a view showing a mode in which a cell was cultured on a glass substrate patterned with PEG or MPC polymer. As the cell, HeLa cell or a bovine aortic vascular endothelial cell was used, and the cell was cultured in known culture conditions. As a result, adhesion and extension of cells were observed only in the region where the glass substrate was exposed. In particular, the bovine aortic vascular endothelial cell could be cultured for a long period of time over 1 month while maintaining this pattern.

### (1) Immobilization of protein on microchannel chip

As the surface modifying agent, MPC polymer was pattern-immobilized on the substrate of a microchannel chip, and heparin to be modified with an active oxidizing species was pattern-immobilized on the substrate.

As shown in Fig. 16, the boundary region between the MPC polymer and heparin produced on the substrate was treated with hypobromous acid generated by a microelectrode. By doing this, only the PEI/heparin layer is modified, therefore, adsorption of a protein on an undesired region can be prevented by performing patterning of the channel chip with such a substrate surface modifying agent in advance.

### (2) Immunoassay in microchannel chip

Then, as shown in Fig. 17, by using a glass substrate patterned with MPC polymer in advance, a microchannel chip was produced.

Then, an immunoassay was carried out by applying the procedure shown in Example 7 (2) to this microchannel chip.

As a result, fluorescence was detected only in the region which was not coated with MPC polymer, and adsorption of a protein on an undesired region could be prevented as described above. These substrate surface modifying agents are stable for a long period of time, therefore, as shown in Fig. 18, it can be expected that these agents can be applied to multi-cell patterning culture in which various cells are cultured, or long-term pattern culture.

### Industrial Applicability

As described in detail above, according to the invention of this application, a protein can be immobilized at a high reproducibility while preventing the protein from inactivation without resort to a large-scaled apparatus and also the protein can be immobilized even in a microchannel, and moreover, the protein-immobilized regions can be arrayed.

Further, according to the invention of this application, by using a cell adhesive protein as the protein to be immobilized, it is also possible to use a cell as a target and to immobilize the cell in an arbitrary region on a substrate.

## Claims

1. A method of immobilizing a protein, **characterized by** comprising the steps of: forming a protein non-adsorptive surface by laminating a protein non-adsorptive substance with a negative charge on a substrate surface with a positive charge; locally modifying the protein non-adsorptive surface into a protein adsorptive surface; and adsorbing a protein in the locally modified region.

2. The method of immobilizing a protein according to claim 1, wherein the substrate surface with a positive charge is formed with a cationic polymer.

3. The method of immobilizing a protein according to claim 1 or 2, wherein the cationic polymer is polyethylenimine.

4. The method of immobilizing a protein according to any one of claims 1 to 3, wherein the protein non-adsorptive substance with a negative charge is at least one substance selected from the group consisting of a glycosaminoglycan, albumin and fibrinogen.

5. The method of immobilizing a protein according to claim 4, wherein the glycosaminoglycan is at least one substance selected from the group consisting of heparin, a heparin derivative and hyaluronic acid.

6. The method of immobilizing a protein according to any one of claims 1 to 5, wherein the modification of the protein non-adsorptive substrate surface into a protein adsorptive substrate surface is carried out with an active chemical species generated by applying an oxidation potential or an oxidation current to an electrode positioned near the substrate.

7. The method of immobilizing a protein according to claim 6, wherein the active chemical species is an active halogen species generated by oxidizing a halide ion.

8. The method of immobilizing a protein according to claim 7, wherein the active halogen species is hypobromous acid (HOBr) or hypochlorous acid (HOCI).

9. A method of immobilizing a protein, **characterized in that** protein-immobilized regions are arrayed by performing the method of immobilizing a protein according to any one of claims 1 to 8 in a sequential manner.

10. The method of immobilizing a protein according to any one of claims 6 to 9, wherein the protein-immobilized regions are established by arraying and immobilizing a protein non-adsorptive substrate surface modifying agent, which is insensitive to the active chemical species, on the substrate surface.

11. The method of immobilizing a protein according to claim 10, wherein the substrate surface modifying agent is at least either of a polyethylene glycol polymer and a methacryloyloxyethyl phosphorylcholine polymer.

12. A protein chip which is produced by the method of immobilizing a protein according to any one of claims 1 to 11, **characterized in that** a protein non-adsorptive substrate surface is locally modified into a protein adsorptive substrate surface and a protein is locally immobilized.

13. The protein chip according to claim 12, wherein a microchannel and an electrode system are embedded in the substrate.

14. The protein chip according to claim 13, wherein the embedded electrode system is a bipolar electrode system comprising one pair of electrodes, and a counter electrode is made of platinum.

15. The protein chip according to any one of claims 12 to 14, wherein the locally immobilized protein is an antibody.

16. The protein chip according to any one of claims 12 to 14, wherein the locally immobilized protein is Protein A or Protein G, and by binding an antibody to this Protein A or Protein G, the antibody is immobilized on the substrate surface.

17. The protein chip according to any one of claims 12 to 14, wherein the locally immobilized protein is an enzyme.

18. The protein chip according to any one of claims 12 to 14, wherein the locally immobilized protein is a cell adhesive protein.

19. The protein chip according to claim 18, wherein the cell adhesive protein is at least one protein selected from the group consisting of fibronectin, collagen and laminin.

20. A method of immobilizing a cell, **characterized by** allowing a cell to adhere on the protein chip according to claim 18 or 19.

21. A cell chip, which is produced by the method of immobilizing a cell according to claim 20, **characterized in that** a cell is locally immobilized thereon.
